# EUROPÄISCHE PATENTANMELDUNG

(11) **EP 0 704 456 A1**
(43) Veröffentlichungstag der Anmeldung: **03.04.1996**
(21) Anmeldenummer: 95111040.2
(22) Anmeldetag: 14.07.1995
(51) Int. Cl.: C07H 15/04

(54) **Verfahren zur Bleichung von Tensidlösungen**

(30) Priorität: 14.09.1994 DE 4432621
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur Bleichung von wäßrigen Tensidlösungen mit Wasserstoffperoxid, wobei eine gezielte Zersetzung des nicht abreagierten Wasserstoffperoxids bzw. daraus gebildeter oxidierend wirkender Folgeprodukte mit Hilfe von Enzymen erfolgt. Die Reaktion erfolgt unter sehr milden Bedingungen und führt zu extrem hellen, geruchsarmen Produkten.

## Beschreibung

Die Erfindung betrifft ein Verfahren zur Bleichung von wäßrigen Tensidlösungen mit Wasserstoffperoxid, wobei in einem ersten Verfahrensschritt die eigentliche Bleichung bei alkalischen pH-Werten in Gegenwart anorganischer Zusatzstoffe, die als Zersetzungsinhibitoren für Wasserstoffperoxid wirken, durchgeführt wird und in einem zweiten Verfahrensschritt eine gezielte Zersetzung des nicht abreagierten Wasserstoffperoxids bzw. daraus gebildeter oxidierend wirkender Folgeprodukte mit Hilfe von Enzymen erfolgt.

Das Verfahren ist insbesondere interessant für Tensidlösungen, die herstellungsbedingt Verfärbungen enthalten, welche vor der Anwendung in Formulierungen für das Gebiet Waschen und Reinigen inklusiv der Personal-Care-Anwendungen zu hellfarbigen Produkten gebleicht werden müssen. Dies trifft insbesondere z. B. auf Alkylpolyglycoside (APG) zu.

Alkylpolyglycoside werden aus natürlichen Rohstoffen hergestellt und sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen. Die Alkylpolyglycoside im Sinne dieser Erfindung genügen der Formel

R' - O - Z_{n'}

in der R' für einen linearen oder verzweigten, gesättigten oder ungesättigten aliphatischen Alkylrest mit 8 bis 18 Kohlenstoffatomen oder Gemische davon und Zₙ für einen Polyglycosylrest mit n = 1,1 bis 3 Hexose- oder Pentoseeinheiten oder Gemische davon stehen.

Bevorzugt werden Alkylpolyglycoside mit Alkylresten mit 12 bis 16 Kohlenstoffatomen sowie einem Polyglycosylrest mit n = 1,1 bis 2. Besonders bevorzugt werden Alkylpolyglycoside mit einem Polyglycosylrest mit n = 1,1 bis 1,4.
Als Polyglycosylrest wird der Polyglucosylrest bevorzugt.

Alkylpolyglycoside mit langkettigen Alkylgruppen werden im allgemeinen durch ein- oder mehrstufige Synthesen hergestellt. Ein einstufiges Herstellverfahren wird u. a. in DE-A-41 01 252 beschrieben.

Ein zweistufiges Herstellverfahren wird beispielsweise in EP-A-0 306 652 angegeben, wonach man zunächst durch Glycosidierung mit n-Butanol ein n-Butylglycosid und darauf durch Umglycosidierung mit einem langkettigen Alkohol das gewünschte langkettige Alkylpolyglycosid herstellt.

Nach beendeter Reaktion liegen die Alkylpolyglycoside in langkettigen Alkoholen gelöst vor. Anschließend müssen diese Alkohole abgetrennt werden, wenn man in Wasser klar lösliche Produkte erhalten will.

Die so erhaltenen wäßrigen APG-Lösungen sind für hohe ästhetische Anforderungen noch zu dunkel und müssen in der Regel einer Bleichung unterzogen werden.

In der Literatur gibt es zahlreiche Hinweise, wie die Bleichung durchgeführt werden kann. So beschreibt Staley (EP 0 165 721) ein Verfahren zur Bleichung mit Wasserstoffperoxid, Schwefeldioxid, Ozon bzw. Persäuren. Dadurch, daß keinerlei Kontrolle bzw. Einstellung des pH-Werts vorgesehen ist, sind die Bleichergebnisse unbefriedigend. Außerdem können so in unerwünschtem Ausmaß Nebenprodukte entstehen, die z. T. auch einen ausgeprägten Eigengeruch haben.

Weitere Bleichmethoden, wie katalytische Hydrierung (US 4 762 918, Staley), Umsetzung mit Borhydrid (EP 0 388 857, Kao) und Bestrahlung mit UV-Licht (EP 0 526 710, Hüls) erwiesen sich als zu wenig wirksam.

Die Bleichung in Gegenwart von Bleichboostern, wie z. B. Erdalkaliionen bzw. Alkalisilicaten (WO 93 13 113, Henkel) unter Verwendung von Wasserstoffperoxid, liefert zwar deutlich bessere Bleichresultate, jedoch ist es schwierig, unter diesen Bedingungen wasserstoffperoxidfreie Produkte zu erhalten. Restmengen an Oxidationsmitteln sind auf jeden Fall zu vermeiden, da sie u. a. die Zersetzung von Zusatzstoffen in APG-haltigen Formulierungen bewirken. Der Vorschlag, das Problem der Rest-Wasserstoffperoxid-Bleichung in Gegenwart von Übergangsmetallverbindungen zu lösen (DE 42 18 073, Henkel), kann ebenfalls nicht überzeugen. Die dann beschleunigte katalytische Zersetzung des Wasserstoffperoxids zu Sauerstoff während der Bleichreaktion verringert die verfügbare Menge an Wasserstoffperoxid. So wird das Bleichergebnis spürbar verschlechtert und uneffektiver.

Es bestand daher die Aufgabe, ein möglichst einfaches Verfahren zu finden, durch das in konzentrierten Tensidlösungen bei der Bleichung nicht abreagiertes Wasserstoffperoxid bzw. daraus gebildete oxidierend wirkende Folgeprodukte im geforderten Maße zersetzt werden.

Gegenstand der Erfindung ist daher ein Verfahren zur Bleichung von konzentrierten wäßrigen Tensidlösungen, dadurch gekennzeichnet, daß in einem ersten Schritt eine oxidierende Bleichung mit Wasserstoffperoxid als Bleichmittel in Gegenwart von Stabilisatoren erfolgt und gegebenenfalls nach einer zwischengeschalteten Peroxid-Vorzersetzung mit Hilfe von Übergangsmetallen und/oder Übergangsmetallverbindungen auf einem Katalysatorbett in einem zweiten Schritt überschüssiges, nicht abreagiertes Bleichmittel und/oder daraus entstandene oxidierend wirkende Folgeprodukte durch Zusatz von katalytisch wirkenden Enzymen beseitigt werden.

Völlig überraschend ist dabei, daß diese enzymatisch katalysierte Reaktion in diesen hochkonzentrierten und damit extrem wasserarmen, d. h. Lösungen mit geringer Wasseraktivität, und bei den relativ hohen Viskositäten in Gegenwart des Stabilisators in so hervorragender Weise ausschließlich in der gewünschten Weise abläuft.

Der erste Verfahrensschritt der Bleichung, z. B. einer Alkylpolyglycosid-Lösung, erfolgt vorzugsweise kontinuierlich im Rührreaktor, im Rohrreaktor, in einer Kombination daraus oder in einer Rührkesselkaskade. Es ist jedoch auch möglich, diskontinuierlich in einem Rührreaktor zu bleichen (Batch-Fahrweise).

Bei der Bleichung beträgt die Konzentration der wäßrigen Alkylpolyglycosid-Lösung 30 bis 75 Gewichtsprozent. Die verwendete Wasserstoffperoxidmenge beträgt 0,1 bis 7, vorzugsweise 0,5 bis 4 Gewichtsprozent (bezogen auf Trockensubstanz). Die Temperatur bei der Bleichung beträgt 40 bis 95 °C, wobei Temperaturen von 50 bis 80 °C besonders bevorzugt werden. Der pH-Wert beträgt 7 bis 12, vorzugsweise 8 bis 11, und die Konzentration an Stabilisierungsmittel (Zersetzungsinhibitor) 50 bis 10 000, vorzugsweise 200 bis 6 000 ppm (bezogen auf die Trockensubstanz). Als Stabilisierungsmittel für das Wasserstoffperoxid werden vorzugsweise anorganische Magnesiumverbindungen eingesetzt.

Der zweite Verfahrensschritt bei der Bleichung beinhaltet die vollständige Zersetzung des überschüssigen Wasserstoffperoxids und/oder der daraus entstandenen oxidierend wirkenden Folgeprodukte mit Enzymen als Katalysator.

Die enzymatisch katalysierte Zersetzung erfolgt nach Zugabe des Enzyms vorzugsweise kontinuierlich unter Durchleiten des Stoffstroms durch einen Rührreaktor, einen Rohrreaktor, einer Kombination daraus oder einer Rührkesselkaskade. Es ist auch möglich, die katalysierte Zersetzung nach der Enzymzugabe im Produktlagerbehälter durchzuführen. Es ist weiterhin auch möglich, nur die Nachreaktion zur Vervollständigung des Abbaus des Wasserstoffperoxids und seiner Folgeprodukte im Produktlagerbehälter durchzuführen. Der enzymatisch katalysierten Zersetzung kann eine andersartige Peroxid-Zersetzung vorgeschaltet sein (z. B. mit Hilfe von Übergangsmetallen und/oder Übergangsmetallverbindungen auf einem Katalysator-Festbett).

Die Temperatur bei der enzymatischen Reaktion beträgt 10 bis 80 °C, vorzugsweise 20 bis 60 °C, und besonders bevorzugt 30 bis 50 °C, je nach verwendetem Enzym.

Der pH-Wert wird auf Werte von 7 bis 12 eingestellt, wobei pH-Werte von 7 bis 10 bevorzugt werden.

Die Enzymmenge hängt von der Enzymaktivität ab. Sie kann von 0,1 bis 5 000 ppm (bezogen auf die Lösungen) betragen, wobei Werte zwischen 1 und 2 000 ppm bevorzugt sind. Es kann zweckmäßig sein, das Enzym an einer oder auch an mehreren Stellen in die Peroxid-Zersetzungsstufe einzudosieren.

Als Beispiele für Enzyme seien insbesondere Katalasen, Glucose-Oxidasen und Superoxid-Dismutasen genannt.
Die Enzyme können auch in stabilisierter oder immobilisierter Form verwendet werden.

Das Verfahren besitzt folgende Vorteile:
- die Peroxid-Zersetzung erfolgt unter milden Bedingungen (keine Gefahr der Produktschädigung),
- die Zersetzungsreaktion ist gut beherrschbar (kein starkes Schäumen durch anfangs zu intensive Sauerstoff-Entwicklung),
- es genügen bei entsprechenden Reaktionsbedingungen kurze Verweilzeiten für die vollständige Peroxid-Zersetzung,
- es entstehen sehr geruchsarme Produkte mit extrem heller Farbe,
- die Produkte sind sicher Wasserstoffperoxid- und Peroxid-frei,
- die Produkte haben bei Erwärmen eine hohe Farbstabilität,
- das Verfahren ist allgemein anwendbar auf herstellungsbedingt dunkel gefärbte Tensidlösungen, wie z. B. von anionischen Tensiden (Beispiele: Paraffinsulfonate, α-Sulfofettsäuremethylester) und nichtionischen Tensiden (Beispiel: APG).

Der ersten und/oder zweiten Stufe des Bleichprozesses kann eine kontinuierlich arbeitende Entschäumermaschine nachgeschaltet sein, in der das u. U. schaumdurchsetzte APG durch Zentrifugieren verdichtet und so besser pumpbar gemacht wird.

## Patentansprüche

1. Verfahren zur Bleichung von konzentrierten, wäßrigen Tensidlösungen,
dadurch gekennzeichnet,
daß in einem ersten Schritt eine oxidierende Bleichung mit Wasserstoffperoxid als Bleichmittel in Gegenwart von Stabilisatoren erfolgt und gegebenenfalls nach einer zwischengeschalteten Peroxid-Vorzersetzung mit Hilfe von Übergangsmetallen und/oder Übergangsmetallverbindungen auf einem Katalysatorbett in einem zweiten Schritt überschüssiges, nicht abreagiertes Bleichmittel und/oder daraus entstandene oxidierend wirkende Folgeprodukte durch Zusatz von katalytisch wirkenden Enzymen beseitigt werden.

2. Verfahren zur Bleichung von wäßrigen Tensidlösungen nach Anspruch 1,
dadurch gekennzeichnet,
daß die Tenside nichtionische Tenside sind.

3. Verfahren zur Bleichung von wäßrigen Tensidlösungen nach Anspruch 1,
dadurch gekennzeichnet,
daß die Tenside Alkylpolyglycoside sind.

4. Verfahren zur Bleichung von wäßrigen Tensidlösungen nach Anspruch 1,
dadurch gekennzeichnet,
daß als Stabilisatoren 50 bis 10 000 ppm (bezogen auf Tensid) anorganische Magnesiumverbindungen verwendet werden.

5. Verfahren zur Bleichung von wäßrigen Tensidlösungen nach Anspruch 1,
dadurch gekennzeichnet,
daß als Enzyme 0,1 bis 5 000 ppm (bezogen auf Tensid) Katalasen verwendet werden.

6. Verfahren Zur Bleichung von wäßrigen Tensidlösungen nach Anspruch 1,
dadurch gekennzeichnet,
daß als Enzyme 0,1 bis 5 000 ppm (bezogen auf Tensid) Glucose-Oxidasen verwendet werden.

7. Verfahren zur Bleichung von wäßrigen Tensidlösungen nach Anspruch 1,
dadurch gekennzeichnet,
daß als Enzyme 0,1 bis 5 000 ppm (bezogen auf Tensid) Superoxid-Dismutasen verwendet werden.

8. Verfahren zur Bleichung von wäßrigen Tensidlösungen nach Anspruch 1,
dadurch gekennzeichnet,
daß die Enzyme in stabilisierter oder immobilisierter Form verwendet werden.

9. Verfahren zur Bleichung von wäßrigen Tensidlösungen nach Anspruch 1,
dadurch gekennzeichnet,
daß die Temperatur bei der enzymatischen Zersetzung 10 bis 80 °C, vorzugsweise 20 bis 60 °C und besonders bevorzugt 30 bis 50 °C, beträgt.

10. Verfahren zur Bleichung von wäßrigen Tensidlösungen nach Anspruch 1,
dadurch gekennzeichnet,
daß der pH-Wert bei 7 bis 12, vorzugsweise bei 7 bis 10, liegt.
